# EUROPEAN PATENT SPECIFICATION

(11) **EP 3 363 471 B1**
(45) Date of publication and mention of the grant of the patent: **23.11.2022**
(21) Application number: 16855487.1
(22) Date of filing: 13.10.2016
(51) Int. Cl.: H05H 1/48

(54) **STERILIZATION DEVICE AND METHOD WITH THE SAME**
STERILISATIONSVORRICHTUNG UND VERFAHREN MIT DIESER
DISPOSITIF DE STÉRILISATION ET PROCÉDÉ AVEC LE MÊME

(30) Priority: 13.10.2015 JP 2015202313; 13.10.2015 JP 2015202314; 25.12.2015 JP 2015253724
(43) Date of publication of application: 22.08.2018
(73) Proprietor: Suntory Holdings Limited, Osaka-shi, Osaka 530-8203 (JP)
(72) Inventor: HIGASHIYAMA, Kenichi, Kyoto 619-0284 (JP); TOMINAGA, Kenta, Kyoto 619-0284 (JP); HIRAYAMA, Yuji, Kyoto 619-0284 (JP); YOSHIHARA, Kazuki, Kyoto 619-0284 (JP)
(74) Representative: Mewburn Ellis LLP
(86) International application number: PCT/JP2016/080430
(87) International publication number: WO 2017/065234

(56) References cited:
- EP-A1- 1 375 357
- DE-A1-102006 050 876
- JP-A- H11 206 860
- JP-A- 2006 331 763
- JP-A- 2007 145 407
- JP-A- 2007 269 325
- JP-A- 2008 237 047
- JP-A- 2012 101 825
- JP-A- 2013 094 468
- JP-A- 2014 050 479
- US-A1- 2013 319 460

## Description

### TECHNICAL FIELD

The present invention relates to sterilization apparatus. More particularly, the present invention relates to sterilization apparatus in which sterilization treatment is carried out by irradiating reactive oxygen, and a sterilization method using the apparatus.

### BACKGROUND ART

In general, many apparatuses usable in the sterilization of foods, medicaments or the like are constituted by a metal such as stainless steel or aluminum, from the viewpoint of corrosion resistance and durability.

For example, in food sterilization apparatus described in Patent Publication 1 the sterilization is carried out by allowing high-temperature, high-pressure steam jetted from a nozzle to enter through previously opened holes, and the nozzle for the steam jetting is constituted of stainless steel.

In Patent Publication 2, when a wrapped food in which a food is filled and tightly sealed in a container such as a cup container or a bag-shaped container is thermally sterilized by microwaves, heated and pressurised air is blown from a pipe into an outer box made of stainless steel at 130° to 150°C and about 3,000 hPa to carry out microwave irradiation of an interior of the wrapped food.

On the other hand, containers for foods or beverages (foodstuffs) or the like are required to be sterilized on internal and external sides thereof. As a conventional sterilization method, methods using aqueous hydrogen peroxide or a chemical have been known. However, there are some disadvantages in that the aqueous hydrogen peroxide or chemical is likely to remain, so that the development of substitute techniques has been studied.

For example, Patent Publication 3 discloses a method including generating a plasma jet using discharge in a fluid, contacting a surface to be treated with the plasma jet and carrying out sterilization (disinfection) by way of energy transfer from the plasma jet to the surfaces. The plasma jet used in this publication is generated by atmospheric electric discharge in a process gas containing oxygen, preferably air, and the nozzle for irradiating this plasma jet is also made of metal.

Patent Publication 4 discloses a cleaning apparatus comprising at least one plasma source designed to ignite at least one plasma in at least one gas and to generate at least one reactive gas.

Patent Publication 5 relates to an insecticidal disinfection sterilizer which uses plasma.

### RELATED ART REFERENCES

### PATENT PUBLICATIONS

Patent Publication 1: Japanese Patent Laid-Open No. 2014-97004
Patent Publication 2: Japanese Patent Laid-Open No. 2010-189034
Patent Publication 3: Japanese Unexamined Patent Publication No. 2009-519799
Patent Publication 4: US Patent Application No. 2013/0319460
Patent Publication 5: Japanese Unexamined Patent Publication No. 2008-237047

### SUMMARY OF THE INVENTION

### PROBLEMS TO BE SOLVED BY THE INVENTION

In general, reactive oxygen species (ROS) such as superoxide radical (•O₂⁻), hydrogen peroxide (H₂O₂), or hydroxy radical (HO•) are produced mainly from oxygen molecules or water in the air. For example, it has been known that hydroxy radicals are obtained by a reaction of water molecules with plasma. Also, these reactive oxygens exhibit an excellent sterilization action due to their strong oxidizing action, and it is considered that the mechanisms thereof are such that sterilization effects are exhibited by the reaction of bacteria existing on the surface based on electron reactivity.

However, in the sterilization method of Patent Publication 3, the patent publication merely discloses a method of mixing a disinfectant substance in the process gas of the source at which the plasma jet is generated in order to enhance the effects (see [0025] of Patent Publication 3), and further techniques have been demanded.

An object of the present invention is to provide a sterilization apparatus having excellent sterilization effects and a sterilization method using the apparatus.

### MEANS TO SOLVE THE PROBLEMS

Accordingly, the first aspect of the present invention provides a sterilisation apparatus according to claim 1. Moreover, as defined in claim 6, a second aspect of the present invention provides a sterilisation method using the sterilisation apparatus of the first aspect of the invention.

Preferred embodiments of the present invention are defined in the dependent claims.

### EFFECTS OF THE INVENTION

The sterilization apparatus of the present invention exhibits some excellent effects in that the sterilization effects are excellent. Also, a chemical or the like which has been used in conventional sterilization does not remain because the sterilization is carried out with a fluid, which leads to simplifications of processing steps, whereby productivity can be remarkably improved.

### BRIEF DESCRIPTION OF THE DRAWINGS

[FIG. 1] FIG. 1 is a schematic view showing one embodiment of an overview of a sterilization apparatus of the present invention.
[FIG. 2] FIG. 2 is schematic views of a sterilization apparatus A-1 which is not part of the invention but it is useful for its understanding, which are a left side view (upper left view), a plan view (lower left view), and a front view (right view) of the irradiation platform. The arrow in the figure shows the direction of progress of an object to be sterilized.
[FIG. 3] FIG. 3 is schematic views of a sterilization apparatus A-2 which is not part of the invention but it is useful for its understanding, which are a left side view (upper left view), a plan view (lower left view), and a front view (right view) of the irradiation platform. The arrow in the figure shows the direction of progress of an object to be sterilized.
[FIG. 4] FIG. 4 is schematic views of a sterilization apparatus A-3 which is not part of the invention but it is useful for its understanding, which are a left side view (upper left view), a planar view (lower left view), and a front view (right view) of the irradiation platform. The arrow in the figure shows the direction of progress of an object to be sterilized.
[FIG. 5] FIG. 5 is schematic views showing a sterilization apparatus of an embodiment A-4 of the present invention, which are a left side view (upper left view), a plan view (lower left view), and a front view (right view) of the irradiation platform. The arrow in the figure shows the direction of progress of an object to be sterilized.
[FIG. 6] FIG. 6 is schematic views showing a sterilization apparatus of an embodiment A-5 of the present invention, which are a left side view (upper left view), a plan view (lower left view), and a front view (right view) of the irradiation platform. The arrow in the figure shows the direction of progress of an object to be sterilized.
[FIG. 7] FIG. 7 is schematic views showing a sterilization apparatus of an embodiment A-6 of the present invention, which are a left side view (upper left view), a plan view (lower left view), and a front view (right view) of the irradiation platform. The arrow in the figure shows the direction of progress of an object to be sterilized.
[FIG. 8] FIG. 8 is a schematic view showing a sterilization apparatus for use with the present invention.
[FIG. 9] FIG. 9 is a schematic view showing a sterilization apparatus for use with the present invention.
[FIG. 10] FIG. 10 is a schematic view showing an embodiment of a unit used in the aging step.
[FIG. 11] FIG. 11 is a schematic view showing an embodiment of a unit used in the aging step.
[FIG. 12] FIG. 12 is a schematic view showing bacteria-inoculated sites internal and external of a resin bottle cap used in Test Example A-1.
[FIG. 13] FIG. 13 is a schematic view showing bacteria-inoculated sites in a resin bottle cap used in Test Example A-1.
[FIG. 14] FIG. 14 is a schematic view showing the state of an internal side of a resin bottle cap used in Test Example B-1.
[FIG. 15] FIG. 15 is a schematic view showing bacteria-inoculated sites in a resin bottle cap used in Test Example B-1 and Test Example C-1.
[FIG. 16] FIG. 16 is a graph showing the relationship between the aging time and the sterilization value while allowing to stand in Test Example C-1.

### MODES FOR CARRYING OUT THE INVENTION

The sterilization apparatus described herein is a sterilization apparatus comprising a reactive oxygen irradiation unit for irradiating a reactive oxygen and an irradiation platform on which an object to be sterilized is placed, wherein the irradiation platform satisfies one or more selected from:
(A): having a reactive oxygen shielding structure in at least one direction of an upper side, a lower side, or a horizontal direction of the object to be sterilized;
(B): being made of a resin and/or made of a non-metal; and
(C): being set within a closed space.

Hereinafter, sterilization apparatus satisfying (A) will be explained as embodiment A, sterilization apparatus satisfying (B) will be explained as embodiment B, and sterilization apparatus satisfying (C) will be explained as embodiment C. Here, in the present invention, the term "sterile" or "sterilization" means breaking of live bodies of microbes or removal thereof from surfaces to be sterilized, which, for example, includes disinfection, sterilization or sterile filtration.

### Embodiment A

It has been known that the electronic reaction of reactive oxygen species is transient, instantaneously disappearing on the surface of an object to be sterilized. In view of the above, as a result of various studies on sustenance of reactive oxygen in order to further increase sterilization effects, the present inventors have surprisingly found that sterilization actions by reactive oxygen are remarkably increased by arranging a particular shielding structure in an irradiation platform so that the reactive oxygen irradiated to the object to be sterilized is controlled to be naturally diffused while having the same amount of reactive oxygen irradiated, whereby perfecting the present invention of the embodiment A.

In the sterilisation apparatus of embodiment A, in accordance with the invention, a particular shielding structure is arranged on an irradiation platform on which an object to be sterilized is placed. The reasons why the sterilization effects are increased by setting a shielding structure cannot be unconditionally explained, but they are assumed to be as follows. While the oxidizing actions of reactive oxygen disappear instantly, the diffusion of reactive oxygen is controlled by the shielding structure in the present invention, so that steam that remains unreacted during the generation of reactive oxygen from plasma can be simultaneously controlled from being diffused, whereby the steam or condensation formed from the steam would contain even larger amounts of reactive oxygen per unit volume, which in turn increases the amount of reactive oxygen retained per unit volume, thereby remarkably increasing the sterilization effects. However, these assumptions do not limit the present invention thereto.

The sterilisation apparatus of embodiment A includes, in accordance with the invention, apparatuses of the embodiments A- 4 to A-6, depending upon the positions at which the reactive oxygen shielding structure is arranged. The embodiments A-1, A-2, A-3 are not covered by the invention but are useful for its understanding.
Embodiment A-1: A sterilization apparatus comprising a reactive oxygen shielding structure which is arranged in an upper side of an object to be sterilized;
Embodiment A-2: A sterilization apparatus comprising a reactive oxygen shielding structure which is arranged in a horizontal direction of an object to be sterilized;
Embodiment A-3: A sterilization apparatus comprising a reactive oxygen shielding structure which is arranged in a lower side of an object to be sterilized;
Embodiment A-4: A sterilization apparatus comprising a reactive oxygen shielding structure which is arranged in a lower side and in a horizontal direction of an object to be sterilized;
Embodiment A-5: A sterilization apparatus comprising a reactive oxygen shielding structure which is arranged in an upper side and in a horizontal direction of an object to be sterilized; and
Embodiment A-6: A sterilization apparatus comprising a reactive oxygen shielding structure which is arranged in all of an upper side, a lower side and a horizontal direction of an object to be sterilized.

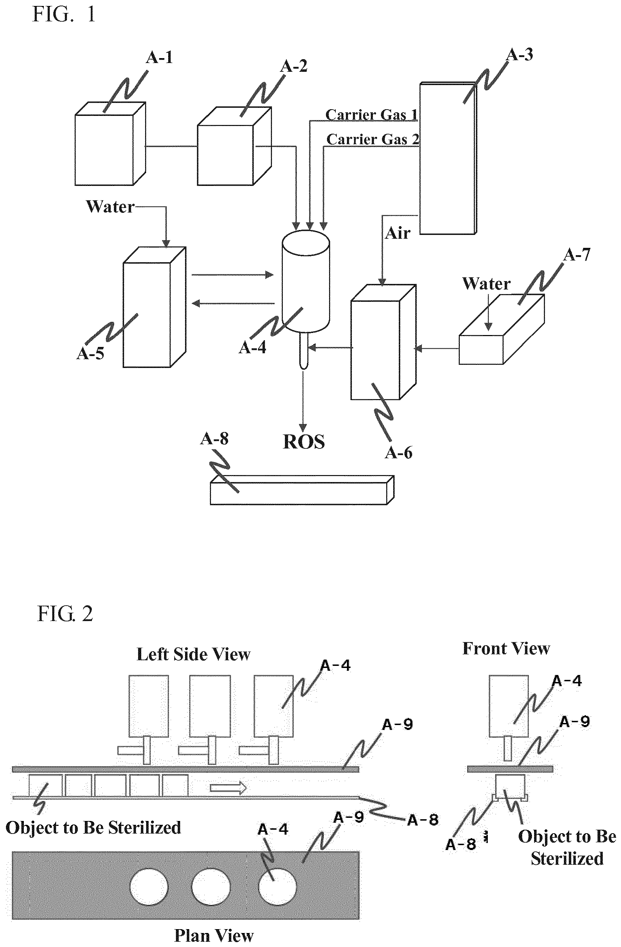

The sterilization apparatuses of the embodiments A-1 to A-6 mentioned above are the same in other constituting elements except for a difference in structures of irradiation platforms, which will be explained more specifically based on FIGs. 1 to 7. Here, FIG. 1 is one example of an overview of the sterilization apparatus of the embodiment A; the present invention is not limited to the embodiments A-4 to A-6 described herein but may take various other forms without departing from the scope of the appended claims. FIGS. 2 to 7 show detailed structures of the irradiation platforms of the embodiments A-1 to A-6.

As shown in FIG. 1, a sterilization apparatus of embodiment A comprises each of the units of an inlet unit A-1 for alternating current, a high-voltage unit A-2, an inlet unit A-3 for gas flow, a nozzle A-4, a chilling unit A-5 of the nozzle, an inlet unit A-6 for steam flow to the nozzle, an inlet unit A-7 for water flow to the inlet unit for steam flow, and an irradiation platform A-8, and a shielding structure A-9 is arranged in the irradiation platform A-8.

The inlet unit A-1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly set in accordance with known techniques. The level of amperes of the alternating current is not particularly limited, and the level can be properly adjusted depending upon the specifications of the inlet device; for example, an alternating current of 11 A may be used. In an embodiment A not being part of the invention, a direct current can be used in place of alternating current, but in the invention alternating current is used because preferred from the viewpoint of adjusting voltage.

The high-voltage unit A-2 is a device which is connected with the inlet unit A-1 for alternating current, and increases voltage of the alternating current supplied from the unit A-1, and any devices that are capable of increasing voltage can be used without particular problems. In addition, the high-voltage unit may be integrated with the unit A-1. The increased voltage is not particularly limited, and may be, for example, from 10 to 30 kV or so.

The inlet unit A-3 for gas flow is an device for inlet of gas flows of various gases to each of a nozzle A-4 and an inlet unit A-6 for steam flow, and a known inlet device for gas flow can be used.

Specifically, a carrier gas for generating plasma is allowed to flow to a nozzle A-4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle A-4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit A-6 for steam flow. By using a water-containing gas in which the steam is mixed with the air, the mixing of the plasma and steam is accelerated, whereby hydroxy radicals can be efficiently produced from the steam. The flow rate of the air to the inlet unit A-6 for steam flow is the same as the flow rate of the water-containing gas to the nozzle A-4. For example, an embodiment of allowing the air to flow at a rate of 3 L/min is exemplified. Here, the air as used herein refers to a gas of which relative humidity is from 0 to 10% by volume or so at 20°C.

The nozzle A-4 is a device for irradiating reactive oxygen obtained by generating plasma, also referred to as a reactive oxygen irradiation unit. The device comprises an internal electrode and an external electrode, and an elevated voltage is applied between the two electrodes from the high-voltage unit A-2, thereby making it possible to generate an electric field. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit A-3 for gas flow, wherein plasma is produced by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit A-6 for steam flow at any of the parts before reaching the discharge opening, at which a reactive oxygen would be produced by a reaction with the plasma produced above, and irradiated from the discharge opening of the reactive oxygen irradiation port.

The nozzle A-4 is not particularly limited in shape or size so long as the nozzle has the above constitution. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit A-5 of the nozzle is a device for allowing chilling water to flow to the nozzle A-4, and a known device for chilling water flow can be used. Since the nozzle A-4 generates heat by applying a high voltage, it is preferable to chill the nozzle. As the chilling water, water at temperature of, for example, 5°C or so are preferably used, and the chilling water may be circulated between the nozzle A-4 and the chilling unit A-5. The flow rate of the chilling water can be properly adjusted so that the surface temperature of the nozzle A-4 is controlled to 25°C or lower. Here, the surface temperature of the nozzle A-4 can be measured with a contact-type thermometer.

The inlet unit A-6 for steam flow to the nozzle is a device of allowing a water-containing gas to flow to the nozzle A-4, and the inlet unit is connected to a reactive oxygen irradiation port of the nozzle A-4 as mentioned above. When the water-containing gas is allowed to flow, first, water from an inlet unit A-7 for water flow is heated with electric heating wires installed therein to produce steam, and a mixture of the steam with the air allowed to flow from the inlet unit A-3 for gas flow is allowed to flow to the nozzle A-4 as a water-containing gas. Here, the inlet unit A-7 for water flow may be integrated with the inlet unit A-6 for steam flow. The heating temperature of the electric heating wires can be properly adjusted depending upon the flow rate of water, which is exemplified by, for example, 300°C. Also, the flow rate of water from the inlet unit A-7 for water flow can be adjusted depending upon the amount of steam needed to produce reactive oxygen. In the present invention, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit A-3 for gas flow in a volume ratio (steam/the air) of from 0.2 to 2.5 or so, and the water-containing air is allowed to flow to a reactive oxygen irradiation port of the nozzle A-4. The mixing volume ratio of steam to the air can be modified by fluctuating the flow rate of water, and the amount of steam contained in the water-containing air can be increased by increasing the flow rate of water. Examples of the mixing volume ratio of the plasma jet produced in the nozzle A-4 to the water-containing gas allowed to flow from the inlet unit A-6 for steam flow [plasma jet / water-containing gas] include from 0.8 to 2.6.

The irradiation platform A-8 on which an object to be sterilized is placed is not particularly limited so long as the object to be sterilized can be placed, and varies only in the locations at which a reactive oxygen shielding structure A-9 is arranged. In the present invention the irradiation platform is arranged on a conveying rail. In addition, it is preferable that the object can be placed at a temperature equal to or lower than an ordinary temperature (40°C), from the viewpoint of allowing hydroxy radicals not to decompose due to high temperatures.

The reactive oxygen shielding structure A-9 (simply referred to as a shielding structure) is arranged as defined in claim 1, and the shielding structure may be in a shape that can inhibit natural diffusion of the reactive oxygen. The shielding structure in each embodiment will be explained using FIGs. 2 to 7.

In the embodiment of FIG. 2 (embodiment A-1), which is not part of the present invention but it is useful for its understanding, a shielding structure is placed at an upper side of objects to be sterilized. Specifically, for example, a shielding structure is placed or connected near a discharge opening of a reactive oxygen irradiation port of the nozzle A-4, in which a plate-like shielding plate can be arranged having a cut-through hole that makes a passageway for the reactive oxygen. The size of the shielding plate can be properly adjusted depending upon the size of an object to be sterilized. It is preferable that the size is such that, for example, when an object to be sterilized is placed on an irradiation platform, the shielding plate has an area which is the same as or greater than a projected area thereof from an upper side, from the viewpoint of confining the sterilization gas in as small a space as possible and increasing an existing amount of the sterilization gas per unit volume. In addition, the size would differ depending upon whether the sterilization is carried out according to a batch process or a continuous process, and, for example, in an apparatus for sterilization according to a continuous process while conveying in one direction, it can be appropriately set according to a known technique so as to cover an upper surface of an object to be sterilized along the direction of progress. Here, the thickness is not particularly limited. Also, it is preferable that the size of the hole is the same as a discharge opening of a reactive oxygen irradiation port, from the viewpoint of confining a sterilization gas in a small space as possible and increasing an existing amount of the sterilization gas per unit volume.

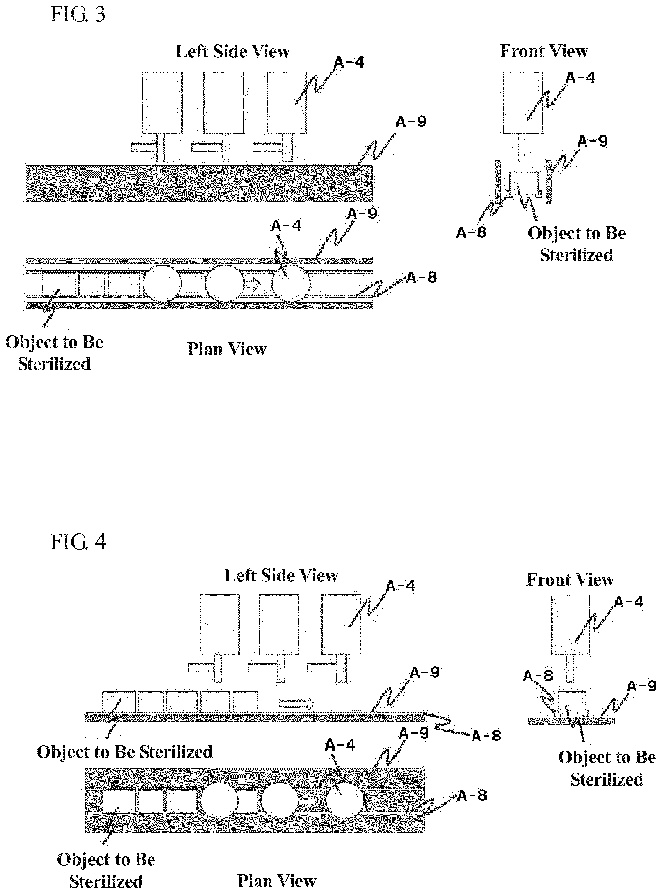

In the embodiment of FIG. 3 (embodiment A-2), which is not part of the present invention but it is useful for its understanding, a shielding structure is placed in a horizontal direction, i.e. a side surface direction, relative to an object to be sterilized. Here, the horizontal direction would differ depending upon whether the sterilization is carried out in a batch process or a continuous process, and in a case of a batch process, the horizontal direction means a direction so as to surround a part or all of the surroundings which are side surfaces of an object to be sterilized, and in a case of a continuous process, the horizontal direction means a wall surface continuously shielding side surfaces extending in the direction of progress. Specifically, for example, when an object to be sterilized is sterilized continuously, a plate-like shielding wall is placed or connected at one side of the irradiation platform, preferably both sides, along the direction of progress. The size of the shielding wall cannot be unconditionally determined depending upon the size of the object to be sterilized, and it is preferable that the size is such that when an object to be sterilized is placed on an irradiation platform, the shielding wall has an area which is equal to or greater than a projected area thereof from a side surface, from the viewpoint of confining the sterilization gas in as small a space as possible and increasing an existing amount of the sterilization gas per unit volume, and the wall has a height which is higher than the height of an object to be sterilized, and preferably having a height 1 to 2 times the height of an object to be sterilized. Here, the thickness is not particularly limited.

In the embodiment of FIG. 4 (embodiment A-3), which is not part of the present invention but it is useful for its understanding, a shielding structure is placed at a lower side of the objects to be sterilized. Specifically, for example, a plate-like shielding plate is placed or connected at a lower side of the irradiation platform on which an object to be sterilized is placed. The size of the shielding plate cannot be unconditionally determined depending upon the size of the object to be sterilized, and it is preferable that the size may be such that the shielding plate has a size on which an object to be sterilized can be placed, from the viewpoint of confining a sterilization gas in a small space as possible and increasing an existing amount of the sterilization gas per unit volume. For example, the shielding plate can be appropriately set in accordance with a known method so that a bottom side of a conveyor belt on which an object to be sterilized is placed can be covered. Here, the thickness is not particularly limited.

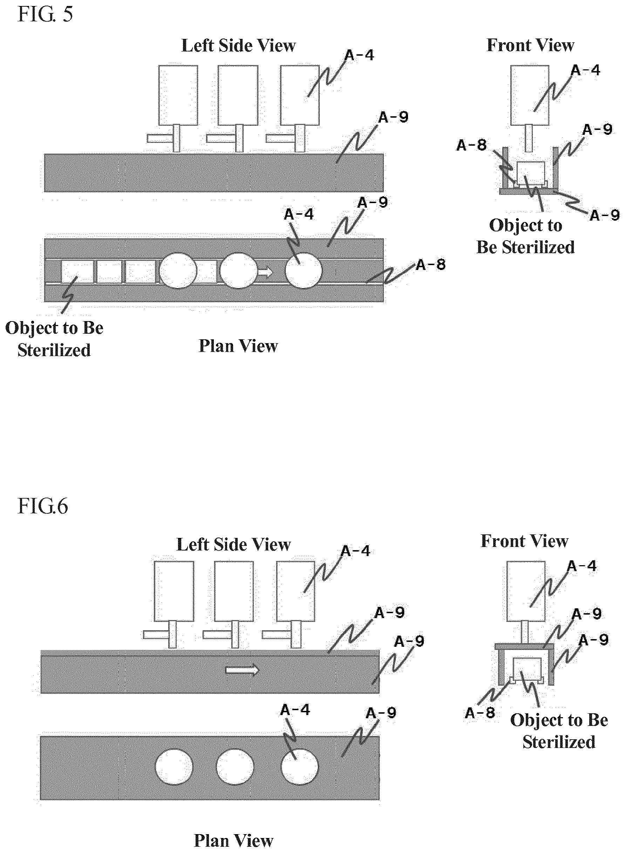

In the embodiment of the present invention of FIG. 5 (embodiment A-4) a shielding structure is placed at a lower side and in a horizontal direction relative to an object to be sterilized. Specifically, the shielding structure is a structure in which shielding structures of embodiment A-2 and embodiment A-3 are combined, and those structures may be connected. For example, as is clear from a front view of FIG. 5, the shielding structure may take a structure that surrounds the object except that an opening is located at an upper side, and which may be an indented structure or groove-like structure. In the above structure, the sterilization effects are more remarkably improved because the sustaining of reactive oxygen is even greater.

In the embodiment of the present invention FIG. 6 (embodiment A-5) a shielding structure is placed at an upper side and in a horizontal direction relative to an object to be sterilized. Specifically, the shielding structure is a structure in which the shielding structures of embodiment A-1 and embodiment A-2 are combined, and those structures may be connected. For example, as is clear from a front view of FIG. 6, the shielding structure may take a structure that surrounds the object except that an opening is located at a lower side, and which may be a cover-like structure or a dome-shaped structure. In the above structure, the sterilization effects are more remarkably improved because the diffusion of reactive oxygen to the exterior is inhibited.

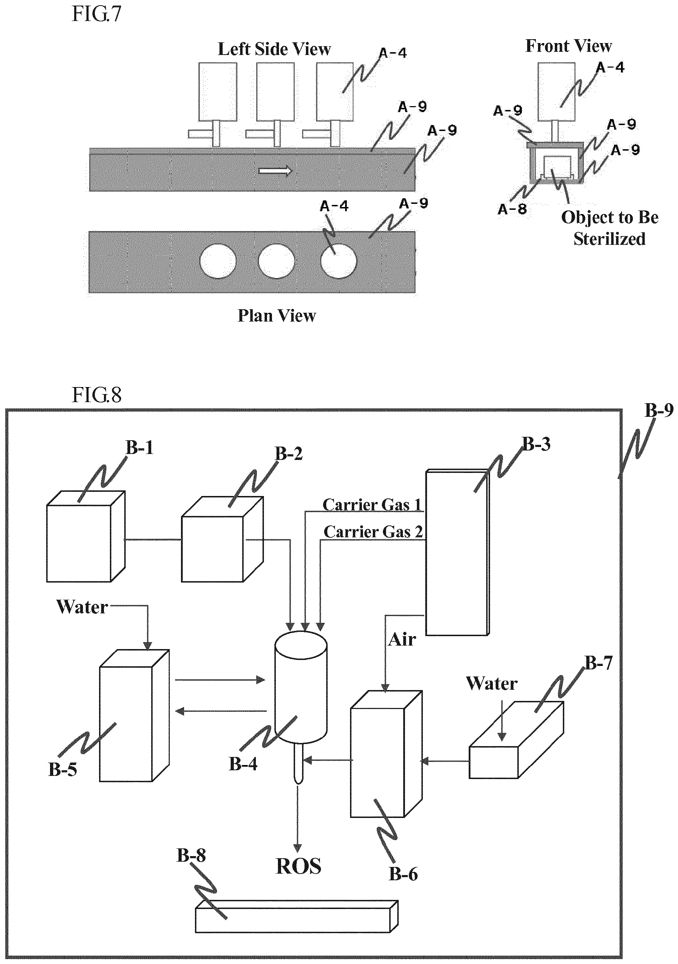

In the embodiment the present invention of FIG. 7 (embodiment A-6) a shielding structure is placed at each one of an upper side, a lower side and a horizontal direction of an object to be sterilized. Specifically, the shielding structure is a structure in which the shielding structures of embodiment A-1, embodiment A-2 and embodiment A-3 are combined, and those structures may be connected. For example, as is clear from a front view of FIG. 7, the shielding structure may take a structure that surrounds all the environment of the object with the shielding structure, and which includes structures like a box with a cover. In this structure sterilization effects are more remarkably improved because the diffusion of reactive oxygen to the exterior is inhibited.

The shielding structure is set as described above, and in embodiment A it is preferable that the shielding structure be made of a resin and/or made of a non-metal, from the viewpoint of even more improving sterilization effects. Here, the matter that the shielding structure is made of a resin and/or made of a non-metal intends to embrace not only a case where an entire shielding structure is constituted by a resin or a non-metal, or a resin and a non-metal, but also a case where a part of the shielding structure is constituted by a resin or a non-metal, or a resin and a non-metal, and a case where the surface of the shielding structure is covered with a resin or a non-metal, or a resin and a non-metal. The resin is not particularly limited so long as the resin is a known resin, and the resin is, for example, preferably olefinic resins such as polyethylene (PE), polypropylene (PP), and ethylene-propylene copolymers; copolymers having ethylene as a monomeric component, such as ethylene-vinyl acetate copolymers (EVA), ionomer resins, ethylene-(meth)acrylic acid copolymers, ethylene-(meth)acrylate ester (random, alternating) copolymers; polyesters such as polyethylene terephthalate (PET), polyethylene naphthalate (PEN), and polybutylene terephthalate (PBT); acrylic resins; polyvinyl chloride (PVC); polyurethanes; polycarbonates; polyphenylene sulfide (PPS); amide-based resins such as polyamides (nylon) and wholly aromatic polyamides (aramid); polyether ether ketone (PEEK); polyimides; polyetherimides; polyvinylidene chloride; ABS (acrylonitrile-butadiene-styrene copolymers); cellulose-based resins; silicone resins; fluororesins, and the like. In addition, the non-metal includes ceramics and the like.

Here, the sterilization apparatus of embodiment A may further comprise other known units besides the units mentioned above. In addition, it may be combined with a known chamber.

Thus, reactive oxygen is irradiated to a platform comprising a particular reactive oxygen shielding structure, and thereby hydroxy radicals are more held, which in turn makes it possible to show excellent sterilization activity. Also, since reactive oxygen is a fluid, even a three-dimensional structured object can be sterilized, exhibiting some excellent effects in that residues do not remain on edges or corners.

### Embodiment B

As a result of various studies in order to further increase the sterilization effects of reactive oxygen, the present inventors have found for the first time that the sterilization effects vary depending upon a surface material on which bacteria are present, and the embodiment B has been perfected thereby.

In general, metals such as stainless steel and aluminium have high corrosion resistance and durability so that they are widely used as constituting materials for a sterilization apparatus. On the other hand, the sterilization effects of reactive oxygen are based on its oxidizing strength, so that the present inventors have intensively studied in order to allow the sterilization effects to be exhibited to the fullest. As a result, they have found that the reduction in the sterilization effects is inhibited due to allowing not to lose oxidizing strength of reactive oxygen by the surrounding environment. Specifically, in a sterilization apparatus of a specific embodiment of the embodiment B for use with the present invention, an oxidation reaction of an irradiation platform itself can be also inhibited by further providing a platform made of a resin and/or a non-metal, on which at least an object to be sterilized is placed, so that it is assumed that much of irradiated reactive oxygen are reacted while retaining the oxidizing strength.

The reactive oxygen in embodiment B is not particularly limited, and, for example, reactive oxygen obtained by generating plasma using alternating current, and generating reactive oxygen from the plasma obtained, is usable.

The sterilization apparatus of embodiment B will be explained in detail hereinbelow on the basis of FIG. 8. Here, the sterilization apparatus shown in FIG. 8 is a chamber sterilization apparatus comprising a chamber, which is merely one embodiment of the present invention, without intending to limit the present invention thereto.

As shown in FIG. 8, the chamber sterilization apparatus of the embodiment B comprises each of the units of an inlet unit B-1 for alternating current, a high-voltage unit B-2, an inlet unit B-3 for gas flow, a nozzle B-4, a chilling unit B-5 of the nozzle, an inlet unit B-6 for steam flow to the nozzle, an inlet unit B-7 for water flow to the inlet unit for steam flow, and an irradiation platform B-8 on which an object to be sterilized is placed, and a chamber B-9.

The inlet unit B-1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly set in accordance with known techniques. The level of amperes of the alternating current is not particularly limited, and the level can be properly adjusted depending upon the specifications of the inlet device; for example, an alternating current of 11 A may be used. Here, in another specific embodiment of the embodiment B not part of the present invention, direct current can be used in place of the alternating current, but in the specific embodiment of embodiment B for use with the present invention alternating current is used because preferred from the viewpoint of adjusting voltage.

The high-voltage unit B-2 is a device which is connected with the inlet unit B-1 for alternating current, and increases voltage of the alternating current supplied from the unit B-1, and any of devices that are capable of increasing voltages can be used without particular problems. In addition, the high-voltage unit may be integrated with the unit B-1. The increased voltage is not particularly limited, and may be, for example, from 10 to 30 kV or so.

The inlet unit B-3 for gas flow is an device for inlet of gas flows of various gases to each of a nozzle B-4 and an inlet unit B-6 for steam flow, and a known inlet device for gas flow can be used.

Specifically, a carrier gas for generating plasma is allowed to flow to a nozzle B-4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle B-4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit B-6 for steam flow. By using a water-containing gas in which the steam is mixed with the air, the mixing of the plasma and steam is accelerated, whereby hydroxy radicals can be efficiently produced from the steam. The flow rate of the air to the inlet unit B-6 for steam flow is the same as the flow rate of the water-containing gas to the nozzle B-4. For example, an embodiment of allowing the air to flow at a rate of 3 L/min is exemplified. Here, the air as used herein refers to a gas of which relative humidity is from 0 to 10% by volume or so at 20°C.

The nozzle B-4 is a device for irradiating reactive oxygen obtained by generating plasma, and is also referred to as a reactive oxygen irradiation unit. The device comprises an internal electrode and an external electrode, and an elevated voltage is applied between both the electrodes from the high-voltage unit B-2, whereby making it possible to generate. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit B-3 for gas flow, wherein plasma is produced by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit B-6 for steam flow at any of the parts before reaching the discharge opening, at which a reactive oxygen would be produced by a reaction with the plasma produced above, and irradiated from the discharge opening of the reactive oxygen irradiation port.

The nozzle B-4 is not particularly limited in the shape or size so long as the nozzle comprises the above parts. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit B-5 of the nozzle is a device for allowing a chilling water to flow to the nozzle B-4, and a known device for chilling water flow can be used. Since the nozzle B-4 generates heat by applying a high voltage, it is preferable to chill the nozzle. As the chilling water, waters at temperatures of, for example, 5°C or so are preferably used, and the chilling water may be circulated between the nozzle B-4 and the chilling unit B-5. The flow rate of the chilling water can be properly adjusted so that the surface temperature of the nozzle B-4 is controlled to 25°C or lower. Here, the surface temperature of the nozzle B-4 can be measured with a contact-type thermometer.

The inlet unit B-6 for steam flow to the nozzle is a device of allowing a water-containing gas to flow to the nozzle B-4, and the inlet unit is connected to a reactive oxygen irradiation port of the nozzle B-4 as mentioned above. When the water-containing gas is allowed to flow, first, water from an inlet unit B-7 for water flow is heated with electric heating wires installed therein to produce steam, and a mixture of the steam with the air from the inlet unit B-3 for gas flow is allowed to flow to the nozzle B-4 as a water-containing gas. Here, the inlet unit B-7 for water flow may be integrated with the inlet unit B-6 for steam flow. The heating temperature of the electric heating wires can be properly adjusted depending upon the flow rate of water, which is exemplified by, for example, 300°C. Also, the flow rate of water from the inlet unit B-7 for water flow can be adjusted depending upon the amount of steam needed to produce reactive oxygen. In the embodiment B, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit B-3 for gas flow in a volume ratio (steam/the air) of from 0.2 to 2.5 or so, and the water-containing air is allowed to flow to a reactive oxygen irradiation port of the nozzle B-4. The mixing volume ratio of steam to the air can be modified by fluctuating the flow rate of water mentioned above, and the amount of steam contained in the water-containing air can be increased by increasing the flow rate of water. Examples of the mixing volume ratio of the plasma jet produced in the nozzle B-4 to the water-containing gas from the inlet unit B-6 for steam flow [plasma jet / water-containing gas] include from 0.8 to 2.6.

The specific embodiment of the embodiment B for use with the present invention has a feature that an irradiation platform B-8 on which an object to be sterilized is placed is made of a resin and/or made of a non-metal. Here, the matter that the irradiation platform is made of a resin and/or made of a non-metal intends to embrace not only a case where an entire irradiation platform is constituted by a resin or a non-metal, or a resin and a non-metal, but also a case where a part of the irradiation platform is constituted by a resin or a non-metal, or by a resin and a non-metal, and a case where the surface of the irradiation platform is covered with a resin or a non-metal, or with a resin and a non-metal. The resin is not particularly limited so long as the resin is a known resin, and the resin is, for example, preferably a resin having ozone resistance. Specific examples include olefinic resins such as polyethylene (PE), polypropylene (PP), and ethylene-propylene copolymers; copolymers having ethylene as a monomeric component, such as ethylene-vinyl acetate copolymers (EVA), ionomer resins, ethylene-(meth)acrylic acid copolymers, ethylene-(meth)acrylate ester (random, alternating) copolymers; polyesters such as polyethylene terephthalate (PET), polyethylene naphthalate (PEN), and polybutylene terephthalate (PBT); acrylic resins; polyvinyl chloride (PVC); polyurethanes; polycarbonates; polyphenylene sulfide (PPS); amide-based resins such as polyamides (nylon) and wholly aromatic polyamides (aramid); polyether ether ketone (PEEK); polyimides; polyetherimides; polyvinylidene chloride; ABS (acrylonitrile-butadiene-styrene copolymers); cellulose-based resins; silicone resins; fluororesins, and the like. In addition, the non-metal includes ceramics and the like. In the present invention, the above resin and the non-metal may be used in other units, among which a nozzle B-4 having a part made of a resin and/or made of a non-metal is preferred.

The temperature of the irradiation platform is not particularly limited, so long as the object to be sterilized can be placed, and it is preferable that the object can be placed at a temperature of equal to or lower than an ordinary temperature (40°C), from the viewpoint of allowing hydroxy radicals not to decompose due to high temperatures.

The chamber B-9 may take an embodiment comprising in its internal at least a nozzle B-4 and an irradiation platform B-8 out of the above units, and the size and the structure thereof can be appropriately set depending upon an object to be sterilized. In addition, it is preferable that the constituting member of the chamber is entirely or partly made of a resin and/or made of a non-metal, in the same manner as in the irradiation platform, from the viewpoint of loss of oxidizing strength due to reaction with hydroxy radicals. The temperature inside the chamber is not particularly set, and the temperature is, for example, from 2° to 40°C.

Here, the chamber sterilization apparatus of the embodiment B may further comprise other units besides the units mentioned above. Examples of other units include shielding walls for preventing diffusion of reactive oxygen, and the like.

Thus, reactive oxygen is irradiated from the chamber sterilization apparatus of the specific embodiment of embodiment for use with the present invention an irradiation platform made of a resin and/or made of a non-metal, so that a loss of oxidizing strength is reduced, which in turn makes it possible to show excellent sterilization activity. Also, since reactive oxygen is a fluid, even a three-dimensional structured object can be sterilized, exhibiting some excellent effects that residues do not remain on edges or corners.

### Embodiment C

The sterilization apparatus of embodiment C has a feature that the irradiation platform is set within a closed space. While reactive oxygen exhibits sterilization actions for the first time by contacting the reactive oxygen with a surface of an object to be sterilized, the inventors of the present application have surprisingly found that when an irradiated object is allowed to stand in the absence of irradiation with reactive oxygen, the sterilization actions are remarkably increased as compared to a time point immediately after the irradiation. Although the detailed mechanisms therefor are unknown, they are assumed as follows. There are some bacteria that undergo instantaneous death and other bacteria remain undead but are only damaged, and when those damaged bacteria are allowed to stand for a certain period of time, the bacteria become fragile and eventually lead to death, thereby making them even more perfectly sterile. As described above, the matter of irradiating reactive oxygen and allowing to stand, thereby increasing the sterilization effects is described as "aging."

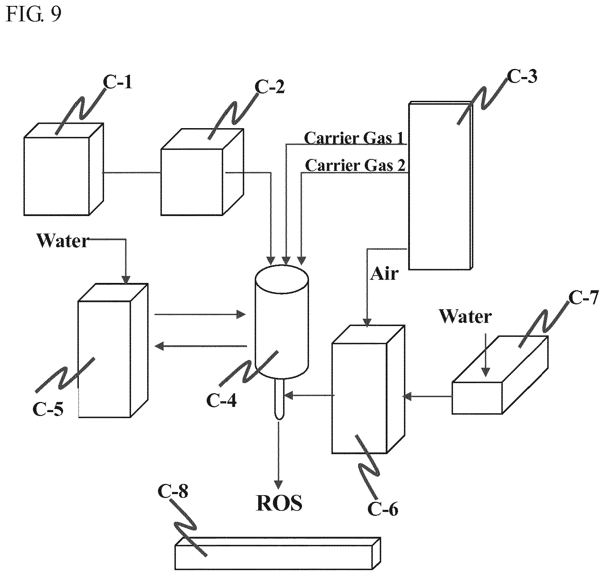

The sterilization apparatus of embodiment C will be explained in detail hereinbelow on the basis of FIG. 9. Here, the sterilization apparatus shown in FIG. 9 is merely one embodiment of the embodiment

As shown in FIG. 9, the sterilization apparatus of embodiment C comprises each of the units of an inlet unit C-1 for alternating current, a high-voltage unit C-2, an inlet unit C-3 for gas flow, a nozzle C-4, a chilling unit C-5 of the nozzle, an inlet unit C-6 for steam flow to the nozzle, an inlet unit C-7 for water flow to the inlet unit for steam flow, and an irradiation platform C-8.

The reactive oxygen in embodiment C is not particularly limited, and, in accordance with a specific embodiment of embodiment C for use with the present invention reactive oxygen obtained by generating plasma using an alternating current, and generating reactive oxygen from the plasma obtained is used.

The inlet unit C-1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly adjusted in accordance with known techniques. The level of amperes of the alternating current is not particularly limited, and the level can be properly adjusted depending upon the specifications of the inlet device; for example, an alternating current of 11 A may be used. In a specific embodiment of embodiment C not being part of the present invention, direct current can be used in place of alternating current, but alternating current is used in a specific embodiment of embodiment C for use with the present invention because preferred from the viewpoint of adjusting voltage.

The high-voltage unit C-2 is a device which is connected with the inlet unit C-1 for alternating current, and increases voltage of the alternating current supplied from the unit C-1, and any devices that are capable of increasing voltages can be used without particular problems. In addition, the high-voltage unit may be integrated with the unit C-1. The increased voltage is not particularly limited, and may be, for example, from 10 to 30 kV or so.

The inlet unit C-3 for gas flow is an device for inlet of gas flows of various gases to each of a nozzle C-4 and an inlet unit C-6 for steam flow, and a known inlet device for gas flow can be used.

Specifically, a carrier gas for generating plasma is allowed to flow to a nozzle C-4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle C-4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit C-6 for steam flow. By using a water-containing gas in which the steam is mixed with the air, the mixing of the plasma and steam is accelerated, whereby hydroxy radicals can be efficiently produced from the steam. The flow rate of the air to the inlet unit C-6 for steam flow is the same as the flow rate of the water-containing gas to the nozzle C-4. For example, an embodiment of allowing the air to flow at a rate of 3 L/min is exemplified. Here, the air as used herein refers to a gas of which relative humidity is from 0 to 10% by volume or so at 20°C.

The nozzle C-4 is a device of irradiating reactive oxygen obtained by generating plasma, which is also referred to as a reactive oxygen irradiation unit. The device comprises an internal electrode and an external electrode, and an elevated voltage is applied between both the electrodes from the high-voltage unit C-2, whereby making it possible to generate. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit C-3 for gas flow, wherein plasma is produced by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit C-6 for steam flow at any of the parts before reaching the discharge opening, at which a reactive oxygen would be produced by a reaction with the plasma produced above, and irradiated from the discharge opening of the reactive oxygen irradiation port.

The nozzle C-4 is not particularly limited in the shape or size so long as the nozzle comprises the above parts. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit C-5 of the nozzle is a device for allowing a chilling water to flow to the nozzle C-4, and a known device for chilling water flow can be used. Since the nozzle C-4 generates heat by applying a high voltage, it is preferable to chill the nozzle. As the chilling water, waters at temperatures of, for example, 5°C or so are preferably used, and the chilling water may be circulated between the nozzle C-4 and the chilling unit C-5. The flow rate of the chilling water can be properly adjusted so that the surface temperature of the nozzle C-4 is controlled to 25°C or lower. Here, the surface temperature of the nozzle C-4 can be measured with a contact-type thermometer.

The inlet unit C-6 for steam flow to the nozzle is a device of allowing a water-containing gas to flow to the nozzle C-4, and the inlet unit is connected to a reactive oxygen irradiation port of the nozzle C-4 as mentioned above. When the water-containing gas is allowed to flow, first, water from an inlet unit C-7 for water flow is heated with electric heating wires installed therein to produce steam, and a mixture of the steam with the air from the inlet unit C-3 for gas flow is allowed to flow to the nozzle C-4 as a water-containing gas. Here, the inlet unit C-7 for water flow may be integrated with the inlet unit C-6 for steam flow. The heating temperature of the electric heating wires can be properly adjusted depending upon the flow rate of water, which is exemplified by, for example, 180°C. Also, the flow rate of water from the inlet unit C-7 for water flow can be adjusted depending upon the amount of steam needed to produce reactive oxygen. In the embodiment C, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit C-3 for gas flow in a volume ratio (steam/the air) of from 0.2 to 2.5 or so, and the water-containing air is allowed to flow to a reactive oxygen irradiation port of the nozzle C-4. The mixing volume ratio of steam to the air can be modified by fluctuating the flow rate of water, and the amount of steam contained in the water-containing air can be increased by increasing the flow rate of water. Examples of the mixing volume ratio of the plasma jet produced in the nozzle C-4 to the water-containing gas from the inlet unit C-6 for steam flow [plasma jet / water-containing gas] include from 0.8 to 2.6.

It is preferable that as to the irradiation platform C-8 on which an object to be sterilized is placed, it is preferable that the object can be placed at a temperature of equal to or lower than an ordinary temperature (40°C), from the viewpoint of allowing hydroxy radicals to be irradiated without decomposing. In addition, the materials thereof are not particularly limited so long they are known ones, and it is preferable that the irradiation platform is entirely or partly constituted by a resin and/or a non-metal.

Also in a specific embodiment of embodiment C for use with the present invention the irradiation platform C-8 is arranged within a closed space, whereby aging can be carried out. The closed space includes, for example, one in which an irradiation platform is formed by covering an irradiation platform with a shielding wall or the like, and a chamber or the like can be preferably used.

As described above, in the sterilization apparatus of embodiment C, since the reactive oxygen is irradiated to an irradiation platform arranged within a closed space, the action of reactive oxygen is fully exhibited on the surface of an object to be sterilized to make it completely sterile, whereby exhibiting excellent sterilization activity.

Here, a method for sterilization in which excellent sterilization activity is obtained by using a sterilization apparatus of the embodiment C will be explained. Specifically, for example, a method includes a method including irradiating reactive oxygen generated from plasma, and thereafter allowing an irradiated objected to be sterilized to stand for a certain period of time (aging) within a closed space.

The step of irradiating reactive oxygen (an irradiating step of reactive oxygen) is not particularly limited, so long as the reactive oxygen is directly irradiated onto an object to be sterilized in a sterilization apparatus of embodiment C mentioned above. The detailed manner of irradiation can be referred to the section about the sterilization apparatus.

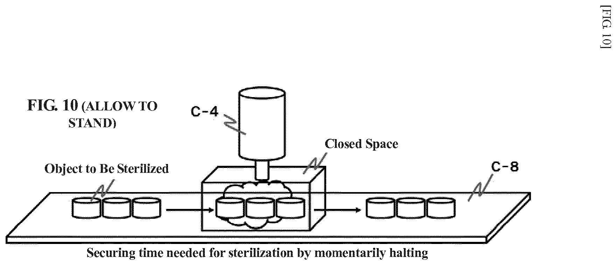

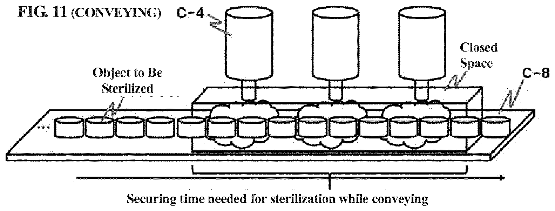

The aging step specifically includes an embodiment including irradiating reactive oxygen, and immediately thereafter carrying out aging while allowing the object to be sterilized to stand within a closed space, and an embodiment including carrying out aging while conveying within a closed space. Accordingly, in the present invention, it is preferable to carry out with a unit comprising an irradiation platform shown in FIG. 10 (an embodiment of allowing to stand) or FIG. 11 (an embodiment of conveying), from the viewpoint of handling the object in a sterile state. The details of the aging step will be explained hereinbelow on the basis of FIGS. 10 and 11.

In FIG. 10, reactive oxygen is irradiated within a closed space, and thereafter allowed to stand for a certain period of time. By irradiating reactive oxygen with a unit as described above, reactive oxygen would be present within a closed space. In accordance with a specific embodiment C of embodiment C for use with the present invention a conveying apparatus is used in such a manner that the irradiation platform is capable of intermittently conveying an object to be sterilized. Specifically, for example, an irradiation platform having a conveyor is paused within a closed space in order to allow an irradiated object to be sterilized to stand beneath a reactive oxygen irradiation port, and after passage for a certain period of time, the conveying is restarted and a fresh object to be sterilized may be conveyed to beneath the reactive oxygen irradiation port. Plural objects to be sterilized may be handled as a single group, and the reactive oxygen may be irradiated while conveying the group. After the termination of irradiation of reactive oxygen to the group, the conveying is halted, and the aging is carried out. The halting time, i.e. the aging time, is at least 4 seconds or longer, preferably 30 seconds or longer, and more preferably 300 seconds or longer. The upper limit is not particularly limited, and it is, for example, 1,800 seconds or so, from the viewpoint of sterilization efficiency.

In FIG. 11, reactive oxygen is irradiated within a closed space, and thereafter aging is carried out while conveying. By irradiating reactive oxygen with a unit as described above, reactive oxygen would be present within a closed space. In accordance with the invention, an irradiation platform in which a conveying device so as to be able to continuously convey an object to be sterilized is used. Specifically, for example, after an irradiation platform having a conveyor or the like which passes through underneath a reactive oxygen irradiation port is irradiated with reactive oxygen, the irradiation platform may be conveyed at a rate so that reactive oxygen would be present within a closed space until a passage of a certain period of time. The time during which the object to be sterilized is detained within the closed space after the reactive oxygen irradiation, i.e. the aging time, is at least 4 seconds or longer, preferably 30 seconds or longer, and more preferably 300 seconds or longer. The upper limit is not particularly limited, and it is, for example, 1,800 seconds or so, from the viewpoint of sterilization efficiency. The rate of the conveying device is properly set in accordance with a known technique so as to give such time.

Here, in embodiment C, it is preferable that aging is carried out while allowing an irradiated object to be sterilized with the reactive oxygen to stand within a closed space or while conveying an irradiated object, but it would also not hamper aging for the object previously discharged from the closed space to be returned into the closed space. In that case, it is preferable that a total of the aging time is within the range mentioned above.

Here, the sterilization apparatus of embodiment C may further comprise other units besides the units mentioned above. Also, in the embodiment C, an apparatus having plural nozzles may be used, and, for example, as shown in FIG. 10 or 11, an apparatus with a constitution in such a manner that nozzles are aligned with the direction of progress of the irradiation platform. In this case, each of the units and tubes is properly arranged so that electric discharging and inlets for gas and steam can be provided to each of the nozzles.

Thus, by using the sterilization apparatus of embodiment C, reactive oxygen within a closed space is irradiated, and aging of an object to be sterilized is carried out, so that bacteria could be sufficiently deadened, thereby making it possible to show excellent sterilization activity. Also, since reactive oxygen is a fluid, even a three-dimensional structured object can be sterilized, thereby exhibiting some excellent effects that residues do not remain on edges or corners.

The present invention is not particularly limited so long as sterilization apparatus of embodiment A, embodiment B or embodiment C mentioned above is is used without departing from the scope of the appended claims. Specific examples of the combination include, for example, in a case of embodiment A and embodiment B, an apparatus in which an irradiation platform comprises a reactive oxygen shielding structure, and is made of a resin and/or made of a non-metal. In addition, in a case of embodiment B and embodiment C, examples not falling within the present invention include an apparatus in which an irradiation platform is arranged within a closed space, and is made of a resin and/or made of a non-metal.

The reactive oxygen to be irradiated in the present invention is warm due to electric discharge within the nozzle or the water-contained gas from the inlet unit for steam flow, the temperature of which is from about 50° to about 80°C. Because of this warmth, the heated load of the irradiated object is considered to be small. Here, the temperature of the reactive oxygen refers to a temperature of reactive oxygen at a discharge opening of the reactive oxygen irradiation port that is measured with a thermocouple thermometer.

In addition, a temperature difference between the reactive oxygen and the surface of the object to be sterilized is, for example, 10°C or more, and more preferably from 25° to 40°C, from the viewpoint of increasing a reactivity of radicals. The temperature of the surface of the object to be sterilized as used herein refers to a temperature of an object to be sterilized that is measured with a contact-type thermometer.

The irradiation speed can be adjusted according to the flow rate of the gas and the shape of the reactive oxygen irradiation port, and, for example, the irradiation speed includes 50,000 mm/sec. The irradiation time is not unconditionally set depending upon the object, and an irradiation time is exemplified by, for example, from 0.05 to 1 second. In the embodiment C, in a case of irradiation from plural nozzles, it is preferable that a total irradiation time is within the above range.

In addition, it is preferable that the distance between the reactive oxygen irradiation port and the surface of the object to be sterilized is, for example, from 5 to 50 mm.

The sterilization apparatus of the present invention is used for irradiating reactive oxygen to an object in need of sterilization. The object is exemplified by, for example, containers for foodstuffs, bottle caps for sealing the opening parts of containers, medical devices, foodstuffs such as vegetables and meat, and the like.

The present invention also provides a sterilization method including carrying out sterilization including irradiating reactive oxygen as defined in claim 6. Here, a method using a sterilization apparatus of the present invention is included, as an apparatus for irradiating the reactive oxygen.

With respect to the sterilization apparatus of embodiment A, there is provided a sterilization method using the apparatus according to claim 1 and carrying out sterilisation as a continuous process. In embodiments not being part of the invention, sterilization method may be a method of sterilization including providing objects to be sterilized in a batch process, and irradiating reactive oxygen thereto to sterilize, or in accordance with the invention is a method of sterilization including providing objects in a continuous process, and irradiating reactive oxygen thereto to sterilize. The conditions for generating reactive oxygen and the specifications and set-up methods of the reactive oxygen shielding structure are in accordance with the section about the sterilization apparatus of embodiments A-4 to A-6.

The sterilization method concerning the sterilization apparatus of the embodiment B, which is not part of the present invention, includes a sterilization method including the following steps (A) and (B):
(A): carrying out environmental sterilization including irradiating reactive oxygen within a chamber, and
(B): carrying out a main sterilization including irradiating reactive oxygen onto the object to be sterilized placed on an irradiation platform made of a resin and/or made of a non-metal located within the chamber to which the sterilization of step (A) was previously carried out.

Here, the reactive oxygen is not particularly limited, and, for example, a reactive oxygen obtained by generating plasma using an alternating current, and generating a reactive oxygen from the plasma obtained is usable.

In the step (A), prior to the sterilization of an intended object by irradiation of reactive oxygen within a chamber, an environmental sterilization within the chamber is carried out. The environmental sterilization refers to cleaning of the environment, and rinsing-off is not needed because of the use of reactive oxygen in the present invention, thereby leading to simplifications in the steps, whereby productivity can be improved.

In step (B) the sterilization is carried out by placing an object to be sterilized on an irradiation platform within a chamber in which the environmental sterilization was previously carried out in the step (A). The specifications and the method of use and the like of the sterilization apparatus are as described in the section of the chamber sterilization apparatus of the embodiment B.

In addition, in the above method, prior to step (A), an embodiment further including a step (A'):
(A'): cleaning an interior of the chamber with an alkali is preferred, from the viewpoint of even more enhancing the effects of the environmental sterilization.

In step (A'), organic substances are removed by cleaning an internal of the chamber with an alkali. As the alkali, NaOH can be preferably used. By carrying out steps (A') and (A) the environmental sterilization would be more effective.

When the above sterilization method is carried out, an embodiment using a chamber sterilization apparatus of embodiment B is preferred.

The sterilization method concerning a sterilization apparatus of embodiment C, which is not part of the present invention, for example, a sterilization method including the steps of:
irradiating a reactive oxygen including irradiating a reactive oxygen generated from plasma to an object to be sterilized; and
aging an irradiated object to be sterilized, thereby carrying out sterilization.

The conditions for generating reactive oxygen and the specifications and the methods of aging are in accordance with the section about the sterilization apparatus of embodiment C.

When the above sterilization method is carried out, an embodiment using sterilization apparatus of embodiment C is preferred.

The sterilization method of the present invention is used for irradiating reactive oxygen to an object in need of sterilization. The object is exemplified by, for example, containers for foodstuff, bottle caps for sealing the opening parts of containers, medical devices, foodstuffs such as vegetables and meat, and the like.

### EXAMPLES

The present invention will be described more specifically by means of Examples given hereinbelow, without intending to limit the present invention thereto.

### TEST EXAMPLE A-1

The influences of the presence or absence of the shielding structure of the irradiation platform in the sterilization apparatus were studied. Specifically, a case where an internal side of a resin bottle cap (material: polyethylene) was inoculated was referred to as an irradiation platform shown in FIG. 5 in which shielding structures are arranged in a lower side and a horizontal side of the object to be sterilized, a case where an external side of the bottle cap was inoculated was referred to as an irradiation platform without a shielding structure, and comparative studies were made therebetween.

### Preparation of Bacterial Solution and Preparation of Bacteria-Inoculated Bottle Cap

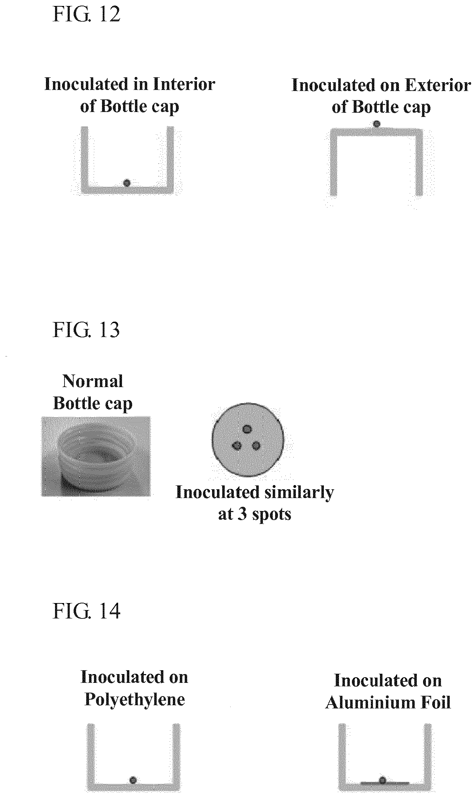

Using a bacterial solution of fibroblast bacterium *Bacillus atrophaeus*, bacterial solutions of various concentrations (3 standards within the concentration range of from 2 × 10³ to 2 × 10⁸ CFU/mL) were prepared. The obtained bacterial solution was inoculated in an internal side or external side of a resin bottle cap as shown in FIG. 12 in an amount of 1 µL × 3 spots for each bottle cap (each concentration n = 5) as shown in FIG. 13. Here, the inoculated resin bottle cap which was allowed to stand in a sterile petri dish for 24 hours to dryness was used.

### Irradiation of Reactive Oxygen

Using sterilization apparatus of the present invention as shown in FIG. 1, a resin bottle cap that was inoculated was irradiated with reactive oxygen for 0.2 seconds per bottle cap from a distance 30 mm upstream side irrespective of the locations of the inoculation on the resin bottle cap, and the irradiated bottle cap was collected on a sterile petri dish. Here, the operating conditions of the sterilization apparatus were as follows. The surface temperature of the bottle cap (a surface temperature of the irradiation platform) was 25°C.

### Operating Conditions of Sterilization Apparatus

Inlet unit A-1 for alternating current: frequency: 14 kHz, voltage: 300 V,
electric current: 11A
High-voltage unit A-2: The raised voltage: 20 kV
Inlet unit A-3 for gas flow: flow rate of air: 6 L/min, flow rate of oxygen: 3 L/min (hereinabove, go to nozzle A-4), flow rate of air: 3 L/min (go to inlet unit A-6 for steam flow)
Nozzle A-4: temperature of reactive oxygen irradiation: 51°C, irradiation speed: 50,000 mm/sec
Chilling unit A-5: chilling water: 5°C
Inlet unit A-6 for steam flow: electric heating wires: 300°C, flow rate of water-containing gas: 4.5 L/min (flow rates of plasma jet/water-containing gas flow (volume ratio) = 9/4.5)
Inlet unit A-7 for water flow: flow rate of water: 1.2 mL/min

### Measurement of Sterilization Activity Values

A resin bottle cap subjected to irradiation of reactive oxygen or a resin bottle cap without subjection to irradiation and allowed to stand in a sterilization apparatus filled with an oxidizing gas was taken out of the sterile petri dish, and 5 mL of TSA liquid medium (manufactured by BD Falcon) was injected to the petri dish, and cultured at 35°C, a temperature suitable for proliferation of microbes for 3 days. After the cultivation, the number of bottle caps in which media became turbid due to microbial proliferation that was judged as positive was counted, and the sterilization activity value LRV (Log Reduction Value) was calculated according to the most probable number method (MPN method). The results are shown in Table 1. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity.

### Table 1

**Table 1**

| | Shielding Structure | LRV, D Value |
|---|---|---|
| Comp. Ex. A-1 | Absent | 2.5 D or less |
| Ex. A-1 | Present | 5.4 D or more |

It can be seen from Table 1 that the sterilization actions are improved in a case where the shielding structure is present. In view of the above, it is suggested that the sterilization actions are improved if the shielding structure or structures arranged to the irradiation platform.

### TEST EXAMPLE B-1

The influences of the materials of the irradiation platform in the sterilization apparatus were studied. Specifically, a case where an internal side of a resin bottle cap (material: polyethylene) was inoculated was referred to as an irradiation platform made of a resin, a case where an internal side of the bottle cap coated with an aluminum foil was inoculated was referred to as an irradiation platform made of a metal, and comparative studies were made therebetween.

### Preparation of Bacterial Solution and Preparation of Bacteria-Inoculated Bottle Cap

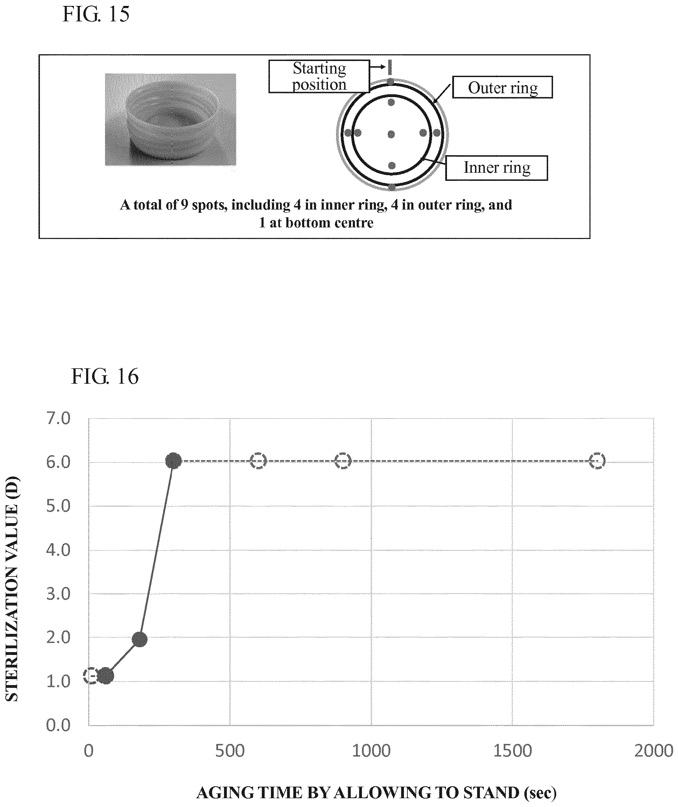

Using a bacterial solution of fibroblast bacterium *Bacillus atrophaeus*, bacterial solutions of various concentrations (3 standards within the concentration range of from 2 × 10³ to 2 × 10⁸ CFU/mL) were prepared. The obtained bacterial solution was inoculated in an internal side of a resin bottle cap as shown in FIG. 14 in an upper side of the aluminum foil or directly in an amount of 1 µL × 9 spots for each bottle cap (each concentration n = 5) as shown in FIG. 15. Here, the inoculated resin bottle cap which was allowed to stand in a sterile petri dish for 24 hours to dryness was used.

### Irradiation of Reactive Oxygen

Using the sterilization apparatus shown in FIG. 8, a resin bottle cap was irradiated with reactive oxygen for 0.2 seconds per bottle cap from a distance 30 mm upstream side, and the irradiated bottle cap was collected on a sterile petri dish. Here, the operating conditions of the sterilization apparatus of the present invention were as follows. A surface temperature of the bottle cap (surface temperature of the irradiation platform) was 25°C, and a temperature inside the chamber B-9 was 28°C.

### Operating Conditions of Sterilization Apparatus

Inlet unit B-1 for alternating current: frequency: 14 kHz, voltage: 300 V, electric current: 11A
High-voltage unit B-2: The raised voltage: 20 kV
Inlet unit B-3 for gas flow: flow rate of air: 6 L/min, flow rate of oxygen: 3 L/min (hereinabove, go to nozzle B-4), flow rate of air: 3 L/min (go to inlet unit B-6 for steam flow)
Nozzle B-4: temperature of reactive oxygen irradiation: 51°C, irradiation speed: 50,000 mm/sec
Chilling unit B-5: chilling water: 5°C
Inlet unit B-6 for steam flow: electric heating wires: 300°C, flow rate of water-containing gas: 4.5 L/min (flow rates of plasma jet/water-containing gas flow (volume ratio) = 9/4.5)
Inlet unit B-7 for water flow: flow rate of water: 1.2 mL/min

### Measurement of Sterilization Activity Values

A resin bottle cap subjected to irradiation of reactive oxygen was taken out of the sterile petri dish, and 5 mL of TSA liquid medium (manufactured by BD Falcon) was injected to the petri dish, and cultured at 35°C, a temperature suitable for proliferation of microbes for 3 days. After the cultivation, the number of the bottle caps in which media became turbid due to microbial proliferation that was judged as positive was counted, and the sterilization activity value LRV (Log Reduction Value) was calculated according to the most probable number method (MPN method). The results are shown in Table 2. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity.

### Table 2

**Table 2**

| | Material of Internal Side of Bottle Cap | LRV, D Value |
|---|---|---|
| Comp. Ex. B-1 | Aluminum Foil | 5.1 D |
| Ex. B-1 | Polyethylene | 5.4 D or more |

It can be seen from Table 2 that the sterilization actions are improved in a case where the material of the internal side of the bottle cap is made of a resin. In view of the above, it is suggested that the sterilization actions are improved if the irradiation platform is made of a resin or made of a non-metal.

### TEST EXAMPLE C-1

The influences of the environment (aging by allowing to stand) of the irradiation platform in the sterilization apparatus were studied.

### Preparation of Bacterial Solution and Preparation of Bacteria-Inoculated Bottle Cap

Using a bacterial solution of fibroblast bacterium *Bacillus atrophaeus*, bacterial solutions of various concentrations (3 standards within the concentration range of from 2 × 10³ to 2 × 10⁸ CFU/mL) were prepared. The obtained bacterial solution was inoculated in a resin bottle cap (material: polyethylene) as shown in FIG. 15 in an amount of 1 µL × 9 spots for each bottle cap (each concentration n = 5). Here, the inoculated resin bottle cap which was allowed to stand in a sterile petri dish for 24 hours to dryness was used.

### Irradiation of Reactive Oxygen

Using the sterilization apparatus shown in FIG. 9 (a conveying portion is shown in FIG. 11), an inoculated resin bottle cap was irradiated with reactive oxygen for 0.5 seconds per bottle cap from a distance 30 mm in an upper side, the irradiated bottle cap was allowed to stand so that the irradiated cap would be present within a closed space for a time as listed in Table 3, and the irradiated bottle cap was then collected on a sterile petri dish. Here, the operating conditions of the sterilization apparatus used were as follows. The surface temperature of the bottle cap (a surface temperature of the irradiation platform) was 25°C.

### Operating Conditions of Sterilization Apparatus

Inlet unit C-1 for alternating current: frequency: 13 kHz, voltage: 350 V, electric current: 11A
High-voltage unit C-2: The raised voltage: 20 kV
Inlet unit C-3 for gas flow: flow rate of air: 6 L/min, flow rate of oxygen: 3 L/min (hereinabove, go to nozzle C-4), flow rate of air: 3 L/min (go to inlet unit C-6 for steam flow)
Nozzle C-4: temperature of reactive oxygen irradiation: 51°C, irradiation speed: 50,000 mm/sec
Chilling unit C-5: chilling water: 5°C
Inlet unit C-6 for steam flow: electric heating wires: 180°C, flow rate of water-containing gas: 4.5 L/min (flow rates of plasma jet/water-containing gas flow (volume ratio) = 9/4.5)
Inlet unit C-7 for water flow: flow rate of water: 1.2 mL/min
Irradiation platform C-8: conveying speed 50 cm/sec

### Measurement of Sterilization Activity Values

A resin bottle cap after a passage of a time allowed to stand listed in Table 3 was taken out of the sterile petri dish, and 5 mL of TSA liquid medium (manufactured by BD Falcon) was injected to the petri dish, and cultured at 35°C, a temperature suitable for proliferation of microbes for 3 days. After the cultivation, the number of bottle caps in which media became turbid due to microbial proliferation that was judged as positive was counted, and the sterilization activity value LRV (Log Reduction Value) was calculated according to the most probable number method (MPN method). The results are shown in Table 3 and FIG. 16. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). The larger the number, the higher the sterilization activity, and the number of 4.5 D or more would show no problem as the sterilization treatment of food containers. In addition, since the results for Comparative Example C-1 and Examples C-2, C-3, and C-4 are estimate values, the values are shown with outlined symbols and broken lines in the graph.

### Table 3

**Table 3**

| | Time Allowed to Stand, sec | LRV, D Value |
|---|---|---|
| Comp. Ex. C-1 | 10 | 1.1 D or less |
| Comp. Ex. C-2 | 60 | 1.1 D |
| Comp. Ex. C-3 | 180 | 2.0 D |
| Ex. C-1 | 300 | 6.0 D |
| Ex. C-2 | 600 | 6.0 D or more |
| Ex. C-3 | 900 | 6.0 D or more |
| Ex. C-4 | 1,800 | 6.0 D or more |

It is suggested that excellent sterilization effects are obtained if the time allowed to stand exceeds 300 seconds.

### INDUSTRIAL APPLICABILITY

The sterilization apparatus of the present invention shows excellent sterilization activity, so that it can be suitably used in, for example, sterilization of containers for foodstuff, bottle caps sealing the openings of the containers, medical devices, foodstuff such as vegetables and meat, and the like.

### EXPLANATION OF NUMERALS

- A-1: inlet unit for alternating current
- A-2: high-voltage unit
- A-3: inlet unit for gas flow
- A-4: nozzle
- A-5: chilling unit
- A-6: inlet unit for steam flow
- A-7: inlet unit for water flow
- A-8: irradiation platform
- A-9: shielding structure
- B-1: inlet unit for alternating current
- B-2: high-voltage unit
- B-3: inlet unit for gas flow
- B-4: nozzle
- B-5: chilling unit
- B-6: inlet unit for steam flow
- B-7: inlet unit for water flow
- B-8: irradiation platform
- B-9: chamber
- C-1: inlet unit for alternating current
- C-2: high-voltage unit
- C-3: inlet unit for gas flow
- C-4: nozzle
- C-5: chilling unit
- C-6: inlet unit for steam flow
- C-7: inlet unit for water flow
- C-8: irradiation platform

## Claims

1. Sterilization apparatus comprising
an alternating current supply unit (A-1);
a high voltage unit (A-2);
a gas flow supply unit (A-3);
a reactive oxygen irradiation unit (A-4);
a chilling unit(A-5);
a steam flow supply unit (A-6);
a water flow supply unit (A-7);
an irradiation platform (A-8);
a conveying rail;
a reactive oxygen shielding structure (A-9);
wherein the high-voltage unit (A-2) is connected to said alternating current supply unit (A-1) and configured to increase the voltage of the alternating current from the alternating current supply unit (A-1);
wherein the reactive oxygen irradiation unit (A-4) is connected to said gas flow supply unit (A-3) through a pipe and comprises
an internal and an external electrode for generating an electric field therebetween by application of the increased voltage from the high-voltage unit (A-2),
a gas inlet port and a reactive oxygen irradiation port with a discharge opening for irradiating reactive oxygen,
wherein the gas inlet port is located at an end of a side opposite to an end part at which the reactive oxygen irradiation port is located;
wherein the chilling unit (A-5) is for allowing chilling water to flow to said reactive oxygen irradiation unit (A-4);
wherein the water flow supply unit (A-7) is configured to supply water to the steam flow supply unit (A-6);
wherein the gas flow supply unit (A-3) is configured to supply one or more carrier gases to said reactive oxygen irradiation unit (A-4) and to supply air to the steam flow supply unit (A-6); wherein the steam flow supply unit (A-6) is connected to said reactive oxygen irradiation unit(A-4), and comprises electrical heating wires configured to produce steam,
said steam flow supply unit (A-6) configured to supply a flow of water-containing gas comprising a mix of said air and said steam to the reactive oxygen irradiation unit (A-4);
the reactive oxygen irradiation unit (A-4) being configured to generate plasma by passing said one or more carrier gases through said electric field and to obtain said reactive oxygen by reacting the generated plasma with said water-containing gas from the steam flow supply unit (A-6);
wherein the irradiation platform (A-8) is for having placed thereon an object to be sterilized by irradiation with the reactive oxygen from the discharge opening of the reactive oxygen irradiation port;
wherein the irradiation platform (A-8) is arranged on said conveying rail for a continuous process;
and wherein the reactive oxygen shielding structure (A-9) is arranged on said irradiation platform to inhibit the diffusion of the reactive oxygen;
wherein the reactive oxygen shielding structure (A-9) is arranged on said irradiation platform such that, during the irradiation of the object to be sterilized, it is placed so as to be:
i) both below and to the side of the object to be sterilized; or
ii) both above and to the side of the object to be sterilized; or
iii) above, below and to the side of the the object to be sterilized.

2. Sterilization apparatus according to claim 1 option ii) or iii), wherein the reactive oxygen shielding structure (A-9) of the irradiation platform comprises a shielding plate having a through-hole as a passageway for the reactive oxygen from the discharge opening.

3. Sterilization apparatus according to claim 1 or 2 in which all or part of the irradiation platform is made of resin and/or of other non-metal.

4. Sterilization apparatus according to any one of the preceding claims in which the reactive oxygen irradiation unit has a part made of resin and/or of other non-metal.

5. Sterilization apparatus according to claim 3 or 4 wherein the resin is an ozone-resistant resin.

6. A sterilization method comprising generating plasma using alternating current and carrying out sterilization as a continuous process comprising irradiating reactive oxygen generated from the plasma obtained onto an object to be sterilised, the method **characterised by** using an apparatus as defined in any one of claims 1-5.

7. A sterilization method of claim 6 in which the object sterilized is selected from containers for foodstuffs, bottle caps for sealing the openings of containers, and medical devices.

## Patentansprüche

1. Sterilisationsvorrichtung, die Folgendes umfasst:
eine Wechselstrom-Versorgungseinheit (A-1);
eine Hochspannungseinheit (A-2);
eine Gasstrom-Zufuhreinheit (A-3);
eine Bestrahlungseinheit für reaktiven Sauerstoff (A-4);
eine Kühleinheit (A-5);
eine Dampfstrom-Zufuhreinheit (A-6);
eine Wasserstrom-Zufuhreinheit (A-7);
eine Bestrahlungsplattform (A-8);
eine Förderschiene;
eine Abschirmstruktur für reaktiven Sauerstoff (A-9);
wobei die Hochspannungseinheit (A-2) mit der Wechselstrom-Versorgungseinheit (A-1) verbunden und ausgelegt ist, um die Spannung des Wechselstroms aus der Wechselstrom-Versorgungseinheit (A-1) zu erhöhen;
wobei die Bestrahlungseinheit für reaktiven Sauerstoff (A-4) mit der Gasstrom-Zufuhreinheit (A-3) durch ein Rohr verbunden ist und Folgendes umfasst:
eine Innenelektrode und eine Außenelektrode, um dazwischen ein elektrisches Feld zu erzeugen, indem die erhöhte Spannung aus der Hochspannungseinheit (A-2) angelegt wird,
eine Gaseinlassöffnung und eine Bestrahlungsöffnung für reaktiven Sauerstoff mit einem Austrittsdurchlass zur Bestrahlung mit reaktivem Sauerstoff,
wobei die Gaseinlassöffnung an einem Ende einer Seite angeordnet ist, die einem Endteil, an dem die Bestrahlungsöffnung für reaktiven Sauerstoff angeordnet ist, entgegengesetzt ist;
wobei die Kühleinheit (A-5) zum Kühlen von Wasser ausgelegt ist, das in die Bestrahlungseinheit für reaktiven Sauerstoff (A-4) strömt;
wobei die Wasserstrom-Zufuhreinheit (A-7) ausgelegt ist, um Wasser zu der Dampfstrom-Zufuhreinheit (A-6) zuzuführen;
wobei die Gasstrom-Zufuhreinheit (A-3) ausgelegt ist, um ein oder mehrere Trägergase zur Bestrahlungseinheit für reaktiven Sauerstoff (A-4) zuzuführen, und um Luft zu der Dampfstrom-Zufuhreinheit (A-6) zuzuführen;
wobei die Dampfstrom-Zufuhreinheit (A-6) mit der Bestrahlungseinheit für reaktiven Sauerstoff (A-4) verbunden ist und elektrische Heizdrähte umfasst, die zur Erzeugung von Dampf ausgelegt sind, wobei die Dampfstrom-Zufuhreinheit (A-6) ausgelegt ist, um einen wasserhältigen Gasstrom, der ein Gemisch aus der Luft und dem Dampf umfasst, zu der Bestrahlungseinheit für reaktiven Sauerstoff (A-4) zuzuführen;
wobei die Bestrahlungseinheit für reaktiven Sauerstoff (A-4) ausgelegt ist, um Plasma zu erzeugen, indem ein oder mehrere Trägergase durch das elektrische Feld geleitet werden, und um den reaktiven Sauerstoff zu erhalten, indem das erzeugte Plasma mit dem wasserhältigen Gas aus der Dampfstrom-Zufuhreinheit (A-6) umgesetzt wird;
wobei die Bestrahlungsplattform (A-8) ausgelegt ist, um ein Objekt darauf zu platzieren, das mittels Bestrahlung mit dem reaktiven Sauerstoff aus dem Austrittsdurchlass der Bestrahlungsöffnung für den reaktiven Sauerstoff sterilisiert werden soll;
wobei die Bestrahlungsplattform (A-8) für einen kontinuierlichen Vorgang auf der Förderschiene angeordnet ist;
und wobei die Abschirmstruktur für reaktiven Sauerstoff (A-9) auf der Bestrahlungsplattform angeordnet ist, um die Ausbreitung des reaktiven Sauerstoffs zu verhindern;
wobei die Abschirmstruktur für reaktiven Sauerstoff (A-9) so auf der Bestrahlungsplattform angeordnet ist, dass sie während der Bestrahlung des zu sterilisierenden Objekts folgendermaßen platziert ist:
i) sowohl unterhalb als auch seitlich des zu sterilisierenden Objekts; oder
ii) sowohl oberhalb als auch seitlich des zu sterilisierenden Objekts; oder
iii) oberhalb, unterhalb und seitlich des zu sterilisierenden Objekts.

2. Sterilisationsvorrichtung nach Anspruch 1 Option ii) oder iii), wobei die Abschirmstruktur für reaktiven Sauerstoff (A-9) der Bestrahlungsplattform eine Abschirmplatte mit einer Durchgangsbohrung als Durchgang für den reaktiven Sauerstoff aus dem Austrittsdurchlass umfasst.

3. Sterilisationsvorrichtung nach Anspruch 1 oder 2, bei der die gesamte oder ein Teil der Bestrahlungsplattform aus Harz und/oder einem anderen Nichtmetall hergestellt ist.

4. Sterilisationsvorrichtung nach einem der vorangegangenen Ansprüche, bei der die Bestrahlungseinheit für reaktiven Sauerstoff einen Teil aufweist, der aus Harz und/oder einem anderen Nichtmetall hergestellt ist.

5. Sterilisationsvorrichtung nach Anspruch 3 oder 4, wobei das Harz ein ozonbeständiges Harz ist.

6. Sterilisationsverfahren, das das Erzeugen von Plasma unter Verwendung von Wechselstrom und die Durchführung einer Sterilisation als kontinuierlichen Vorgang umfasst, der das Bestrahlen eines zu sterilisierenden Objekts mit dem reaktiven Sauerstoff, der aus dem erhaltenen Plasma erzeugt wurde, umfasst, wobei das Verfahren durch die Verwendung einer Vorrichtung nach einem der Ansprüche 1 bis 5 gekennzeichnet ist.

7. Sterilisationsverfahren nach Anspruch 6, wobei das zu sterilisierende Objekt aus Behältern für Lebensmittel, Flaschenkappen zum Verschließen von Behälteröffnungen und medizinischen Vorrichtungen ausgewählt ist.

## Revendications

1. Appareil de stérilisation comprenant
une unité d'alimentation en courant alternatif (A-1) ;
une unité haute tension (A-2) ;
une unité d'alimentation en écoulement gazeux (A-3) ;
une unité d'émission de rayonnement d'oxygène réactif (A-4) ;
une unité de refroidissement (A-5) ;
une unité d'alimentation en écoulement de vapeur (A-6) ;
une unité d'alimentation en écoulement d'eau (A-7) ;
une plateforme d'exposition à un rayonnement (A-8) ;
un rail de transport ;
une structure de blindage d'oxygène réactif (A-9) ;
dans lequel l'unité haute tension (A-2) est reliée à ladite unité d'alimentation en courant alternatif (A-1) et conçue pour accroître la tension du courant alternatif à partir de l'unité d'alimentation en courant alternatif (A-1) ;
dans lequel l'unité d'émission de rayonnement d'oxygène réactif (A-4) est reliée à ladite unité d'alimentation en écoulement gazeux (A-3) par le biais d'un conduit et comprend
une électrode interne et externe pour générer un champ électrique entre elles par application de la tension accrue à partir de l'unité haute tension (A-2),
un orifice d'entrée de gaz et un orifice d'émission de rayonnement d'oxygène réactif avec une ouverture de décharge pour émettre un rayonnement d'oxygène réactif,
dans lequel l'orifice d'entrée de gaz est situé au niveau d'une extrémité d'un côté opposé à une partie d'extrémité au niveau de laquelle est situé l'orifice d'émission de rayonnement d'oxygène réactif ;
dans lequel l'unité de refroidissement (A-5) est destinée à permettre l'écoulement d'eau de refroidissement vers ladite unité d'émission de rayonnement d'oxygène réactif (A-4) ;
dans lequel l'unité d'alimentation en écoulement d'eau (A-7) est conçue pour fournir de l'eau à l'unité d'alimentation en écoulement de vapeur (A-6) ;
dans lequel l'unité d'alimentation en écoulement gazeux (A-3) est conçue pour fournir un ou plusieurs gaz vecteurs à ladite unité d'émission de rayonnement d'oxygène réactif (A-4) et pour fournir de l'air à l'unité d'alimentation en écoulement de vapeur (A-6) ;
dans lequel l'unité d'alimentation en écoulement de vapeur (A-6) est reliée à ladite unité d'émission de rayonnement d'oxygène réactif (A-4), et comprend des câbles chauffants électriques conçus pour produire de la vapeur,
ladite unité d'alimentation en écoulement de vapeur (A-6) conçue pour fournir un écoulement de gaz contenant de l'eau comprenant un mélange dudit air et de ladite vapeur à l'unité d'émission de rayonnement d'oxygène réactif (A-4) ;
l'unité d'émission de rayonnement d'oxygène réactif (A-4) étant conçue pour générer du plasma en faisant passer lesdits un ou plusieurs gaz vecteurs à travers ledit champ électrique et pour obtenir ledit oxygène réactif en faisant réagir le plasma généré avec ledit gaz contenant de l'eau à partir de l'unité d'alimentation en écoulement de vapeur (A-6) ;
dans lequel la plateforme d'exposition à un rayonnement (A-8) est destinée à recevoir sur celle-ci un objet devant être stérilisé par rayonnement avec de l'oxygène réactif à partir de l'ouverture de décharge de l'orifice d'émission de rayonnement d'oxygène réactif ;
dans lequel la plateforme d'exposition à un rayonnement (A-8) est agencée sur ledit rail de transport pour un processus continu ;
et dans lequel la structure de blindage d'oxygène réactif (A-9) est agencée sur ladite plateforme d'exposition à un rayonnement pour empêcher la diffusion de l'oxygène réactif ;
dans lequel la structure de blindage d'oxygène réactif (A-9) est agencée sur ladite plate-forme d'exposition à un rayonnement de telle sorte que, lorsque l'objet devant être stérilisé est exposé à un rayonnement, elle est placée de sorte à être :
i) à la fois au-dessous et sur le côté de l'objet devant être stérilisé ; ou
ii) à la fois au-dessus et sur le côté de l'objet devant être stérilisé ; ou
iii) au-dessus, au-dessous et sur le côté de l'objet devant être stérilisé.

2. Appareil de stérilisation selon l'option ii) ou iii) de la revendication 1, dans lequel la structure de blindage d'oxygène réactif (A-9) de la plateforme d'exposition à un rayonnement comprend une plaque de blindage ayant un trou traversant en tant que passage pour l'oxygène réactif à partir de l'ouverture de décharge.

3. Appareil de stérilisation selon la revendication 1 ou 2 dans lequel l'ensemble ou une partie de la plateforme d'exposition à un rayonnement est composé(e) de résine et/ou d'un autre matériau non métallique.

4. Appareil de stérilisation selon l'une quelconque des revendications précédentes dans lequel l'unité d'émission de rayonnement d'oxygène réactif a une partie composée de résine et/ou d'un autre matériau non métallique.

5. Appareil de stérilisation selon la revendication 3 ou 4 dans lequel la résine est une résine résistante à l'ozone.

6. Procédé de stérilisation comprenant la génération de plasma à l'aide de courant alternatif et la mise en œuvre d'une stérilisation en tant que processus continu comprenant l'émission d'un rayonnement d'oxygène réactif généré à partir du plasma obtenu sur un objet devant être stérilisé, le procédé étant **caractérisé par** l'utilisation d'un appareil tel que défini dans l'une quelconque des revendications 1 à 5.

7. Procédé de stérilisation selon la revendication 6 dans lequel l'objet stérilisé est sélectionné parmi des récipients pour denrées alimentaires, des capsules pour sceller les ouvertures de récipients et des dispositifs médicaux.
